# EUROPÄISCHE PATENTANMELDUNG

(11) **EP 2 455 485 A1**
(43) Veröffentlichungstag der Anmeldung: **23.05.2012**
(21) Anmeldenummer: 10191872.0
(22) Anmeldetag: 19.11.2010
(51) Int. Cl.: C12Q 1/68

(54) **Verfahren zum Nachweis von Nukleinsäuren**

(71) Anmelder: Anagnostics Bioanalysis GmbH, 4300 St. Valentin (AT)
(72) Erfinder: Ronacher, Bernhard, 4020 Linz (AT)
(74) Vertreter: Sonn & Partner Patentanwälte

(57) **Zusammenfassung**

Die vorliegende Erfindung betrifft ein Verfahren zum parallelen Nachweis mindestens eines spezifischen Nukleinsäurezielmoleküls in einer Mehrzahl an Proben mittels Nukleinsäureamplifikation umfassend die Schritte:
a) Bereitstellen von mindestens zwei Nukleinsäure umfassenden Proben,
b) Kontaktieren der Proben aus Schritt a) mit einem Vorwärtsprimer und einem Rückwärtsprimer umfassend einen 3'- und 5'-Abschnitt, wobei der Vorwärts- und der Rückwärtsprimer im 3'-Abschnitt eine zum amplifizierenden Nukleinsäurezielmolekül komplementäre Nukleotidsequenz aufweisen und der Vorwärts- oder der Rückwärtsprimer im 5'-Abschnitt ein Oligonukleotid mit einer künstlichen, den einzelnen Proben zugeordneten Nukleinsäuresequenz umfasst,
c) Amplifizieren der Proben aus Schritt b),
d) Kontaktieren der amplifizierten Proben aus Schritt c) mit einem festen Träger, auf welchem an einem vorselektierten Ort Oligonukleotide immobilisiert sind, welche an deren 3'-Ende die künstliche, den einzelnen Proben zugeordnete Nukleinsäuresequenz umfassen, und
e) Feststellen der Bindung der Amplifikationsprodukte aus Schritt c) an den am festen Träger immobilisierten Oligonukleotiden.

## Beschreibung

Die vorliegende Erfindung betrifft Verfahren zum parallelen Nachweis mindestens eines spezifischen Nukleinsäurezielmoleküls in mindestens zwei Proben.

Bei verschiedenen Hybridisierungs-Methoden, beruhend auf der Ausbildung von DNA Doppelsträngen bei komplementären Sequenzen, können künstliche DNA Sequenzen eingesetzt werden. Diese Sequenzen umfassen meist 10 bis 25 Nukleotide und stimmen mit keiner bekannten natürlichen Sequenz überein. Diese Oligonukleotide können dabei einen zweiten Sequenzteil umfassen. Der 5'-liegende Teil ist der künstliche Teil und der 3'-liegende Teil der natürliche und komplementär zu der Zielsequenz in der Probe. Werden so gestaltete Oligonukleotide in einer Polymerisationsreaktion eingesetzt, sind die entstehenden Amplifikationsprodukte über die eingebauten Oligonukleotide am 5'-Ende über die Sequenz eindeutig zuordenbar. Bei einer nachfolgenden Hybridisierung der erhaltenen Amplifikationsprodukte, werden an der Festphase Oligonukleotide verwendet, die zu den künstlichen Sequenzen im Amplifikationsprodukt komplementär sind. Dadurch können z.B. Punktmutationen nach Primer Extension festgestellt werden (siehe z.B. DE 3807994, US 2003/148284, EP 1 186 669, EP 0 530 794). Die vier dabei eingesetzten Primer unterscheiden sich dabei am 3'-Ende und tragen ein A, T, G oder C und am 5'-liegenden Teil des Primers, da sie jeweils eine völlig unterschiedliche künstliche Sequenz tragen. Mutationsanalysen, die mehrere DNA Basenpaare betreffen, werden auf diese Art technisch unmöglich. Grund ist die Verwendung von sehr hohen Primerkonzentrationen mit sehr ähnlicher Primer-Sequenz (z.B. für ein Triplett müssten Primer für die erste Base [a] (vier) für die zweite Base [b] (4*4) und für die dritte Base [c] (4*4*4) also 4³= 64 Primer in Lösung verwendet werden. Die maximale Anzahl von bestehenden Multiplex PCR Systemen liegt derzeit bei unter 30.

Die bisher bekannten Verfahren zum Nachweis von spezifischen Molekülen in einer Probe umfassen die Schritte der Probeentnahme, Probenaufbereitung, Zielmolekül-Anreicherung (gegebenenfalls inkl. Markierung), Hybridisierung an der Festphase, Waschen und Nachweisreaktion (z.B. Bestimmung der Farbe). Dabei wird eine Hybridisierungseinheit (z.B. eine Glasplatte) benötigt. Für die nächste Probe muss eine neue Hybridisierungseinheit (oder aufwendige Regeneration) verwendet werden. Eine Folge derartiger Verfahren ist, dass dadurch der Durchsatz (hybridisierte Proben pro Zeit) sehr gering ist. Eine Parallelisierung ist mit derartigen Verfahren nicht möglich.

Aufgabe der vorliegenden Erfindung ist es, Verfahren zur Verfügung zu stellen, mit deren Hilfe Moleküle, insbesondere Nukleinsäuremoleküle, spezifisch in einer Probe nachgewiesen werden können.

Daher betrifft die vorliegende Erfindung ein Verfahren zum parallelen Nachweis mindestens eines spezifischen Nukleinsäurezielmoleküls in einer Mehrzahl an Proben mittels Nukleinsäureamplifikation umfassend die Schritte:
a) Bereitstellen von mindestens zwei Nukleinsäure umfassenden Proben,
b) Kontaktieren der Proben aus Schritt a) mit einem Vorwärtsprimer und einem Rückwärtsprimer umfassend einen 3'- und 5'-Abschnitt, wobei der Vorwärts- und der Rückwärtsprimer im 3'-Abschnitt eine zum amplifizierenden Nukleinsäurezielmolekül komplementäre Nukleotidsequenz aufweisen und der Vorwärts- oder der Rückwärtsprimer im 5'-Abschnitt ein Oligonukleotid mit einer künstlichen, den einzelnen Proben zugeordneten Nukleinsäuresequenz umfasst,
c) Amplifizieren der Proben aus Schritt b),
d) Kontaktieren der amplifizierten Proben aus Schritt c) mit einem festen Träger, auf welchem an einem vorselektierten Ort Oligonukleotide immobilisiert sind, welche an deren 3'-Ende die künstliche den einzelnen Proben zugeordnete Nukleinsäuresequenz umfassen, und
e) Feststellen der Bindung der Amplifikationsprodukte aus Schritt c) an den am festen Träger immobilisierten Oligonukleotiden.

Mit dem erfindungsgemäßen Verfahren ist es möglich mehrere spezifische Nukleinsäuren in einer Mehrzahl von Proben parallel in einem einzigen Detektionssystem und einem einzigen Verfahrensschritt zu bestimmen. Dabei werden zunächst die in den Proben nachzuweisenden Nukleinsäuren in einem ersten Verfahrensschritt amplifiziert. Einer der verwendeten Primer (der Vorwärts- oder der Rückwärtsprimer) weist im 5'-Abschnitt eine künstliche Nukleinsäuresequenz auf, die in keinem der Proben vorkommt bzw. nicht in der Lage ist mit dem weiteren Primer ein Amplifikationsprodukt unter Amplifikationsbedingungen zu erzeugen. Die künstliche Nukleinsäuresequenz ist probenspezifisch, d.h. jeder Probe wird eine spezifische Sequenz zugeordnet. Dadurch können die im Zuge der Amplifikation hergestellten Produkte, welche am 5'-Ende die künstliche Nukleinsäuresequenz bzw. am 3'-Ende des Komplementärstranges die komplementäre künstliche Nukleinsäuresequenz aufweisen, an den festen Träger, an welchem Oligonukleotide umfassend die künstliche Nukleinsäuresequenz immobilisiert sind, gebunden werden. Umfasst die Probe somit die Zielsequenz, binden die Amplifikationsprodukte am festen Träger.

Der Schritt des Kontaktierens der amplifizierten Probe mit dem festen Träger erfolgt unter Bedingungen, die eine spezifische Bindung der Amplifikationsprodukte an die am festen Träger immobiliserten Oligonukleotide ermöglichen. Um die Effizienz dieser Bindung zu erhöhen und um unspezifisch gebundene Nukleinsäuremoleküle vom festen Träger zu entfernen, kann der feste Träger nach dem Schritt des Kontaktierens gewaschen werden. Bei den Waschschritten und den Schritten, bei denen die Amplifikationsprodukte an die immobilisierten Oligonukleotide gebunden werden, werden vorzugsweise stringente Bedingungen eingesetzt.

Erfindungsgemäß werden unter dem Begriff "stringente Bedingungen" Bedingungen subsumiert, unter denen eine Nukleinsäuresequenz vorzugsweise an deren Zielsequenz hybridisieren wird, und zu einem geringeren Ausmaß oder gar nicht an andere Sequenzen. Im Allgemeinen werden stringente Bedingungen so ausgewählt, dass die Temperatur etwa 5°C unter dem thermischen Schmelzpunkt (Tₘ) für die spezifische Sequenz bei einer definierten Ionenstärke und einem definierten pH liegt. Die Tₘ ist die Temperatur (unter definierter Ionenstärke, pH und Nukleinsäurekonzentration), bei der 50% der zu der Zielsequenz komplementären Moleküle zu der Zielsequenz im Gleichgewichtszustand hybridisieren. Typischerweise sind stringente Bedingungen solche, bei denen die Salzkonzentration mindestens ungefähr 0,01 bis 1,0 M Natriumionen-Konzentration (oder ein anderes Salz) bei einem pH zwischen 7,0 und 8,3 beträgt und die Temperatur mindestens 30°C für kurze Moleküle (also z. B. 10-50 Nukleotide) beträgt. Zusätzlich können stringente Bedingungen durch Zugabe destabilisierender Agenzien, wie beispielsweise Formamid, erreicht werden.

Geeignete stringente Hybridisierungsbedingungen sind beispielsweise auch beschrieben in Sambrook et al. (Molecular Cloning: A Laboratory Manual, 3. Auflage, Cold Spring Harbor Laboratory Press, Cold Spring Harbor, New York). So kann die Hybridisierung beispielsweise unter den folgenden Bedingungen stattfinden:
Hybridisierungspuffer: 2x SSC, 10x Denhardt's Lösung (Fikoll 400+PEG+BSA; Verhältnis 1:1:1), 0,1% SDS, 5mM EDTA, 50mM Na₂HPO₄, 250µg/ml Heringssperma-DNA; 50µg/ml tRNA oder 0,25M Natriumphosphatpuffer pH 7,2, 1mM EDTA, 7% SDS bei einer Hybridisierungstemperatur von 65°C bis 68°C

Waschpuffer: 0,2x SSC, 0,1% SDS bei einer Waschtemperatur von 65°C bis 68°C

Erfindungsgemäß wird das Verfahren bis Schritt c) einzeln, d.h. getrennt, für jede Probe durchgeführt. Nach Schritt c) können die mit den künstlichen, den einzelnen Proben zugeordneten Nukleinsäuresequenzen "markierten" Amplifikationsprodukte vermischt und deren Anwesenheit parallel bestimmt werden.

Erfindungsgemäß werden mindestens zwei (vorzugsweise mindestens drei, vier, fünf, sechs, sieben, acht, neun, zehn) unabhängige Proben getrennt aufbereitet. Die Nukleinsäuren (DNA oder RNA) werden getrennt voneinander für die Hybridisierung markiert (PCR). Es laufen somit mindestens zwei identische Prozesse parallel ab, die sich dadurch unterscheiden, dass die verwendeten Primer alle den gleichen 3'-liegenden Abschnitt (für das Zielgen), aber eine unterschiedliche 5'-liegende Oligosequenz haben. Die erhaltenen Amplifikate werden nach der Markierungsreaktion (PCR) gemischt und gemeinsam einer Hybridisierungseinheit (festen Träger) zugeführt. Dort erfolgt die Doppelstrangausbildung mit den an der Festphase gebundenen Oligonukleotiden nach den mitgebrachten "Reverse Komplement" Sequenzen (künstlichen Nukleinsäuresequenzen). Daraus folgt, bei erfolgreicher Hybridisierung an einem Oligonukleotid muss auch ein Amplifikatsprodukt entstanden sein. Und ein Amplifikatsprodukt entsteht nur, wenn in der Probe auch ein Target (Zielgen) enthalten war. Findet keine Hybridisierung statt, war die Probe negativ. So können in einem Hybridisierungsschritt mindestens zwei unabhängige Proben parallel analysiert werden, unabhängig davon, ob die einzelnen Proben positiv oder negativ sind. Dieses Prinzip kann auf "beliebig" viele parallel ablaufende Reaktionen erweitert werden. So können z.B. in einer 96 Well Platte, 96 Proben auf ein PCR Ereignis getestet werden und über die eingebrachten Oligonukleotide auf einen einzigen festen Träger gleichzeitig hybridisiert bzw. analysiert werden. Der Durchsatz erhöht sich hierbei um den Faktor 96.

Erfindungsgemäß ist eine "künstliche, den einzelnen Proben zugeordnete Nukleinsäuresequenz" eine Nukleinsäuresequenz, welche in der Probe nicht vorhanden ist bzw. unter stringenten Bedingungen nicht in der Lage ist an ein Nukleinsäuremolekül in der Probe zu binden. Dadurch wird es ermöglicht, dass jedes amplifizierte oder markierte Molekül in der Probe an einen vorselektierten Ort am Träger gebunden werden kann.

Gemäß der vorliegenden Erfindung bedeutet "an einem vorselektierten Ort", dass die erfindungsgemäß verwendeten Oligonukleotide mit den künstlichen, den einzelnen Proben zugeordneten Nukleinsäuresequenzen, je nach Sequenz, an mindestens einen definierten Ort am Träger immobilisiert sind. Werden die Oligonukleotide auf den festen Träger gespottet, umfasst somit jeder einzelne Spot lediglich eine Art von Oligonukleotid, welche einer Probe zugewiesen ist.

Der Träger, auf dem die Oligonukleotide immobilisiert sind, kann jegliche geometrische Form aufweisen, die für vergleichbare Nachweisverfahren geeignet ist. Bevorzugt werden solche Träger eingesetzt, die üblicherweise bei Hybridisierungen verwendet werden. Gemäß einer bevorzugten Ausführungsform der vorliegenden Erfindung ist der Träger planar oder rotationssymmetrisch ausgeformt.

Rotationssymmetrische Träger können besonders vorteilhaft im erfindungsgemäßen Verfahren eingesetzt werden. Daher besteht der rotationssymmetrische Träger vorzugsweise aus einem unter Freilassung eines radialen Ringspaltes in einen Probenbehälter einsetzbaren Rotor, dessen Umfangsfläche mit den Oligonukleotiden immobilisiert ist. Derartige Träger sind in der Fachwelt hinreichend bekannt und beispielsweise in der WO 03/100401 und der WO 2007/041734 eingehender beschrieben.

Gemäß einer bevorzugten Ausführungsform der vorliegenden Erfindung weisen die Oligonukleotide mit der künstlichen, den einzelnen Proben zugeordneten Nukleinsäuresequenz eine Länge von 15 bis 30, vorzugsweise 18 bis 25, noch mehr bevorzugt von 19 bis 23, Basenpaaren auf.

Gemäß einer weiteren bevorzugten Ausführungsform der vorliegenden Erfindung weisen die Oligonukleotide mit der künstlichen, den einzelnen Proben zugeordneten Nukleinsäuresequenz einen Schmelzpunkt zwischen 50 und 70°C, vorzugsweise ungefähr 60°C, auf. Um derartige Nukleinsäuresequenzen zu designen, können herkömmliche Berechnungsverfahren eingesetzt werden. Entsprechende Verfahren sind in der Fachwelt hinreichend bekannt.

Um schließlich festzustellen, ob ein spezifisches Nukleinsäuremolekül (Amplifikationsprodukt) in einer oder mehreren Proben vorhanden ist, muss festgestellt werden, ob im Zuge des Verfahrens ein Nukleinsäuremolekül am festen Träger gebunden wird. Gemäß einer bevorzugten Ausführungsform der vorliegenden Erfindung sind die Rückwärtsprimer mit einem Farbstoff markiert. Durch eine derartige Markierung wird es ermöglicht, die Bindung eines markierten Amplifikationsprodukts am festen Träger festzustellen.

Alternativ dazu ist es erfindungsgemäß auch möglich, die Amplifikationsprodukte nach Schritt c) oder d) mit einem Farbstoff zu markieren. Dabei können zur Amplifikation modifizierte Nukleotide (z.B. fluoreszenzmarkierte Nukleotide) verwendet werden. Eine weitere Möglichkeit ist die Verwendung von Nicht-Farbstoffen. Dazu gehören chemische Gruppen (Moleküle), die ihrerseits Ziel für weitere Interaktionen sind. Ein Beispiel dafür ist Biotin, welches mit markiertem Streptavidin ein messbares Signal erzeugt. Der Farbstoff kann Eigenschaften der Absorption in unterschiedlichen Bereichen des elektromagnetischen Spektrums besitzen. Vorzugsweise im ultravioletten (UV), im visuellen oder Infrarot-Bereich. Der Farbstoff kann aber auch als Partikel wirken. So sind Gold-Partikel oder andersartige Partikel bekannt, die eine Interferenz mit elektromagnetischen Wellen eingehen. Ebenso ist die Verwendung von magnetischen oder paramagnetischen Beads vorstellbar.

Gemäß einer bevorzugten Ausführungsform der vorliegenden Erfindung ist der Farbstoff ausgewählt aus der Gruppe bestehend aus Alexa Fluor, insbesondere Alexa Fluor 350, Alexa Fluor 488, Alexa Fluor 430, Alexa Fluor 555, Alexa Fluor 610 und Alexa Fluor 647, Bodipy (boron-dipyrromethene), insbesondere Bodipy 493/503, Bodipy R6G-X, Bodipy 530/550, Bodipy 558/568, Bodipy 564/570, Bodipy TMR-X, Bodipy 576/589, Bodipy 581/591, Bodipy TR-X, Bodipy 630/650, Bodipy 650/665 und Bodipy FL, Oregon Green, insbesondere Oregon Green 488, Oregon Green 500 und Oregon Green 514, FAM, FITC, Fluorescein, Fluorescein-dT, Rhodamin Green, TET, JOE, Yakima Yellow, HEX, CY3, TAMRA, Rhodamin Red, CY3.5, ROX, Texas Red, CY5, CY5.5, IRD700 und IRD800.

Die am festen Träger gebundenen Amplifikationsprodukte können auch einer Primer-Verlängerung unterzogen werden. Dabei kommt es nach der Hybridisierung an die immobilisierten Primer zu einer Polymerisationsreaktion, vergleichbar mit einer in Flüssigkeit ablaufenden PCR. Das dafür nötige Enzym (Polymerase) wird gleichzeitig oder nach der Hybridisierung zugesetzt. Ebenso die für die Reaktion nötigen Bestandteile im Puffer (dNTPs, Salze und Stabilisatoren). Der Träger wird dabei nach der Hybridisierung auf stringente Bedingungen gebracht und danach auf Polymerisationstemperatur (Taq Polymerase mit 72°C-74°C). Die Polymerisation läuft dann vom immobilisierten Primer solange ab, bis das hybridisierte DNA Molekül als Matrize (Vorlage) dient. Ziel der Primerextension ist, über die Hybridisierung hinaus weitere Informationen über das entstandene Amplifikat zu bekommen und damit über die Probenbestandteile (Sequenz der DNA).

Ob eine Primer Extension stattfand oder nicht, kann durch eine nachgelagerte Schmelzkurve erkannt werden. Die verlängerten Primer und das hybridisierte Amplifikat weisen eine weit höhere Schmelztemperatur auf, da sich die Längen um mehr als das Doppelte unterscheiden. Damit ist ein Unterschied von 10 bis 20°C erreichbar. Nur hybridisierte Primer schmelzen bis zur Erreichung der Amplifkatsschmelztemperatur völlig ab und erzeugen keinerlei Signal am Spot. Verlängerte Primer haben eine verbleibende Signalhöhe, wodurch eine klare Unterscheidung erzielt werden kann. Als Beispiel kann so festgestellt werden, ob in einer Probe ein Gen (DNA Molekül) mit der Primersequenz vorhanden ist und welches Basenpaar als nächstes in der Sequenz folgt.

Mit Hilfe der Primer-Verlängerung können auch modifizierte Targets für eine Shotgun-Sequenzierung hergestellt werden.

Das erfindungsgemäße Verfahren kann auch zur Vorbereitung von Probenmaterial (DNA) für eine nachfolgende Sequenzierung verwendet werden. Shotgun-Sequenzierung basiert auf Seqeuenzierreaktionen von DNA (oder auch Amplifikaten)-Stücken, die an beiden Enden eine gleichartige Sequenz tragen. Diese Sequenz ist in der ursprünglichen DNA nicht vorhanden und muss in einem vorgelagerten Aufbereitungsschritt eingebracht werden. Derzeit werden in einem aufwendigen Verfahren sogenannte Tags (kurze DNA Doppelstrangstückchen) an alle vorliegenden Proben DNA Fragmente ligiert. Dieser Schritt beinhaltet gegebenenfalls auch die Einbringung von Proben kennzeichnenden Nukleinsäuresequenzen innerhalb der Tags. Dieser Ligationsschritt ist ungerichtet und unpräzise. Die Konsequenz daraus ist, dass alle Proben DNA Fragmente Tags tragen und gleichberechtigt in den Sequenzierungsprozess eingeschleust werden. Für die Analyse einer Probe sind jedoch nur die Sequenzdaten von einigen wenigen Fragmenten nötig bzw. gewünscht. Damit ist die Shotgun-Sequenzierung sehr effizient in der Methode der Sequenzierung, aber sehr ineffizient in der Treffsicherheit. Das macht die Methode derzeit sehr ineffizient.

Mit Hilfe des erfindungsgemäßen Verfahrens wird die Anzahl an Fragmenten auf die nötigen Fragmente (zB. Oncogene) reduziert und durch Einbringung von Probenidentifikationssequenzen erleichtert. Proben DNA spezifische Primer werden dabei für eine Primer-Verlängerung verwendet. Diese tragen an ihrem 5'-Ende eine Tag Sequenz und eine zusätzliche künstliche Nukleinsäuresequenz zur Unterscheidung der einzelnen Proben. Wird ein Primer komplementär zum DNA Fragment der Probe verlängert, entsteht ein Template für eine weitere Reaktion. Ein in Flüssigkeit befindlicher Primer kann auf dieses neu entstandene Target binden und seinerseits verlängert werden. Auch dieser Primer trägt ein Tag und die künstliche Nukleinsäuresequenz. Das entstandene Produkt ist nun eine einzelsträngige DNA, die einem definierten Proben DNA Fragment entspricht und an beiden Enden ein künstliches Tag trägt. Es folgt ein Waschen des Trägers bei hoher Temperatur. Auf dem festen Träger verbleiben nur die kovalent gebundenen Primer und die noch höher schmelzenden, neu entstandenen Einzelstrang DNA Moleküle mit dem Tag. Durch Inkubation bei 94°C werden auch diese Nukleinsäuremoleküle vom festen Träger eluiert. Das Eluat dient dann als Eingangsprodukt für die Shotgun-Sequenzierung. Mit dem erfindungsgemäßen Verfahren kann somit die Fragmentzahl auf die Zielgene reduziert werden, die Fragmente tragen Tags und die künstlichen Nukleinsäuresequenzen für die parallele Abarbeitung von mehreren Proben in einem einzigen Sequenzierungslauf. Die Shot-gun Sequenzierungsmethode ist somit sehr effizient und treffsicher.

Ein alternativer, verallgemeinernder Aspekt der vorliegenden Erfindung betrifft ein Verfahren zum parallelen Nachweis mindestens eines spezifischen Moleküls in einer Mehrzahl an Proben umfassend die Schritte:
a) Bereitstellen von mindestens zwei Proben,
b) Kontaktieren der Proben aus Schritt a) mit einem Bindungspartner, welcher spezifisch an das zumindest eine nachzuweisende Molekül bindet, wobei der Bindungspartner an ein Oligonukleotid mit einer künstlichen, den einzelnen Proben zugeordneten Nukleinsäuresequenz gekoppelt ist,
c) Kontaktieren der Proben aus Schritt b) mit einem festen Träger, auf welchem an einem vorselektierten Ort Oligonukleotide immobilisiert sind, welche komplementär zu den am Bindungspartner gekoppelten Oligonukleotiden sind, und
d) Feststellen der Bindung der in der Probe nachzuweisenden Moleküle aus Schritt c) an den am festen Träger immobilisierten Oligonukleotiden.

Das erfindungsgemäße Konzept ist nicht nur zum Nachweis von Nukleinsäuremolekülen in mehreren Proben geeignet. Auch spezifische Moleküle wie Polypeptide und Proteine (z.B. Antikörper) können erfindungsgemäß in mehreren Proben parallel bestimmt werden. Dabei werden die Proben vor dem Inkontaktbringen mit dem festen Träger einzeln mit Bindungspartnern der zu bestimmenden Moleküle vermischt. Die spezifischen Bindungspartner sind mit den Proben zugewiesenen Oligonukleotiden markiert bzw. an diese gekoppelt. Diese Oligonukleotide dienen dazu, die zu bestimmenden Moleküle an einer vorselektierten, der Probe zugewiesenen Stelle am festen Träger zu binden.

Die Bindung eines spezifischen Moleküls am Träger kann beispielsweise durch Zugabe eines zweiten Bindungspartners erfolgen, welcher in der Lage ist an das Molekül spezifisch zu binden. Der zweite Bindungspartner ist dabei vorzugsweise mit einem Farbstoff markiert.

Gemäß einer bevorzugten Ausführungsform der vorliegenden Erfindung ist der Träger planar oder rotationssymmetrisch ausgeformt.

Der rotationssymmetrische Träger besteht vorzugsweise aus einem unter Freilassung eines radialen Ringspaltes in einen Probenbehälter einsetzbaren Rotor, dessen Umfangsfläche mit den Oligonukleotiden immobilisiert ist.

Gemäß einer weiteren bevorzugten Ausführungsform der vorliegenden Erfindung weisen die Oligonukleotide mit der künstlichen, den einzelnen Proben zugeordneten Nukleinsäuresequenz eine Länge von 15 bis 30, vorzugsweise 18 bis 25, noch mehr bevorzugt von 19 bis 23, Basenpaaren auf.

Die Oligonukleotide mit der künstlichen, den einzelnen Proben zugeordneten Nukleinsäuresequenz weisen vorzugsweise einen Schmelzpunkt zwischen 50 und 70°C, vorzugsweise ungefähr 60°C, auf.

Gemäß einer bevorzugten Ausführungsform der vorliegenden Erfindung werden die am festen Träger immobiliserten Moleküle mit einem zweiten Molekül-spezifischen Bindungspartner, welcher mit einem Farbstoff markiert ist, vor Schritt d) in Kontakt gebracht.

Der Farbstoff ist vorzugsweise ausgewählt aus der Gruppe bestehend aus Alexa Fluor, insbesondere Alexa Fluor 350, Alexa Fluor 488, Alexa Fluor 430, Alexa Fluor 555, Alexa Fluor 610 und Alexa Fluor 647, Bodipy (boron-dipyrromethene), insbesondere Bodipy 493/503, Bodipy R6G-X, Bodipy 530/550, Bodipy 558/568, Bodipy 564/570, Bodipy TMR-X, Bodipy 576/589, Bodipy 581/591, Bodipy TR-X, Bodipy 630/650, Bodipy 650/665 und Bodipy FL, Oregon Green, insbesondere Oregon Green 488, Oregon Green 500 und Oregon Green 514, FAM, FITC, Fluorescein, Fluorescein-dT, Rhodamin Green, TET, JOE, Yakima Yellow, HEX, CY3, TAMRA, Rhodamin Red, CY3.5, ROX, Texas Red, CY5, CY5.5, IRD700 und IRD800.

Die vorliegende Erfindung wird weiters anhand der folgenden Figuren und Beispiele näher illustriert, ohne auf diese beschränkt zu sein.

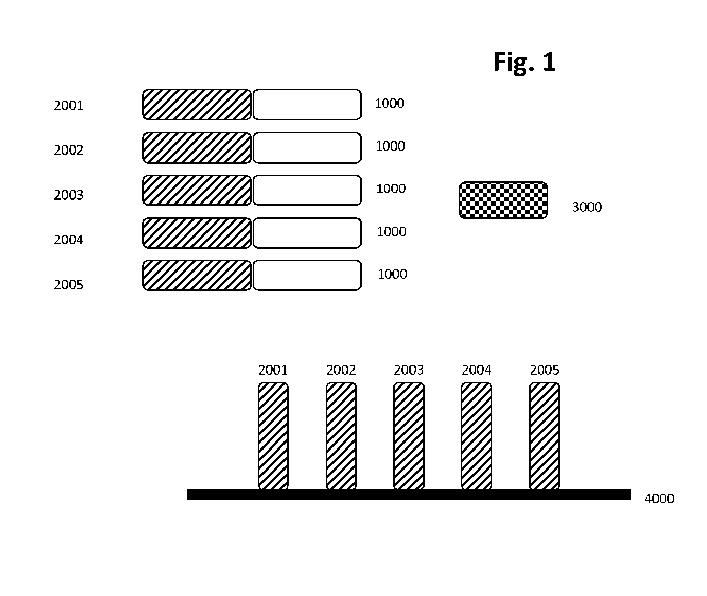

Fig. 1 zeigt illustrativ das Grundprinzip des erfindungsgemäßen Verfahrens. Dabei werden Proben von 5 verschiedenen Patienten auf das Vorhandenseins einer spezifischen Nukleinsäure hin untersucht. Jedem einzelnen Patienten wird dabei eine künstliche Nukleinsäuresequenz zugeordnet. Die verwendeten Vorwärtsprimer (2000) umfassen am 5'-Ende diese künstliche Sequenz (2001-2005). Am 3'-Ende dieser Primer befindet sich ein Abschnitt, der in der Lage ist an das zu amplifizierende Nukleinsäurezielmolekül zu binden (1000). Der Rückwärtsprimer (3000) ist mit einem Farbstoff markiert und für alle Einzelreaktionen ident.

Fig. 1 zeigt zudem die Oberfläche eines festen Trägers (4000), an der die Oligonukleotide immobilisiert sind, welche die den Patienten zugeordneten künstlichen Nukleinsäuresequenzen umfassen. Ein Spot (2011 - 2055) umfasst dabei jeweils eine Art von Oligonukleotid, so dass ein Spot einem Patienten direkt zugeordnet werden kann.

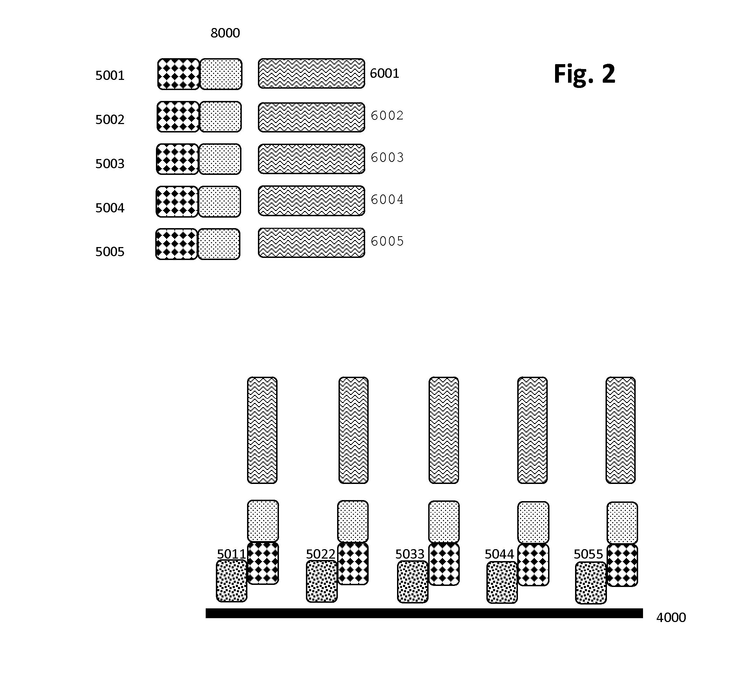

Fig. 2 zeigt einen alternativen Aspekt der vorliegenden Erfindung. Dabei werden fünf Proben, welche ein nachzuweisendes Molekül, z.B. Antikörper (6001 - 6005), umfassen, unabhängig voneinander mit Bindungspartnern (8000) kontaktiert, an welche Proben-spezifische künstliche Oligonukleotide (5001 - 5005) gebunden bzw. gekoppelt sind. Diese Oligonukleotide sind in der Lage an einen festen Träger zu binden, an welchem Oligonukleotide (5011 - 5055) immobilisiert sind, welche eine zu den an den Bindungspartnern gebundenen bzw. gekoppelten Oligonukleotiden komplementäre Nukleinsäuresequenz aufweisen. Da am festen Träger Spots mit einer Art von Oligonukleotiden immobilisert sind, binden die in der Probe vorhandenen Moleküle nur an den vorgesehenen Spot. Die Detektion von am festen Träger gebundenen Molekülen kann beispielsweise durch einen zweiten Farbstoff-markierten Bindungspartner erfolgen (sekundärer Antikörper).

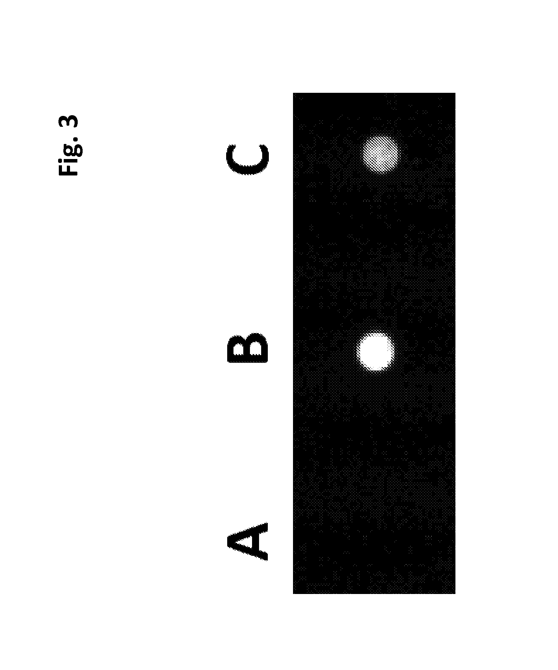

Fig. 3 zeigt einen Scan von drei Spots nach einer protein hybcode Hybridisierung. A Oligo 572 (nicht komplementär); B Protein direkt geprintet und C komplementäres Oligo 571.

Fig. 4 zeigt eine Verdünnungsreihe einer Inkubation von Protein-DNA Oligo Chimäre mit nachfolgender Inkubation mit anti-BSA Antikörper zur Detektion (Fluoreszenz-markiert). A Oligo 572 (nicht komplementär); B Protein direkt geprintet und C komplementäres Oligo 571.

### BEISPIELE:

### Beispiel 1:

Die chemische Kopplung von unterschiedlichen Biomolekülen zueinander ist bekannt. Eine Ausprägung ist die kovalente Verbindung von Protein und DNA (synthetische Oligonukleotide). Dazu können unterschiedliche chemische Reaktionen benutzt werden. Dabei wurde in einem ersten Schritt das Protein mit einer HyNic Gruppe (6-hydrazinopyridine-3-carboxylic acid) chemisch modifiziert. Parallel dazu wurde ein DNA Oligonukleotid mit Aminomodifikation chemisch mit einer 4FB-Gruppe (4-formylbenzamide) modifiziert. In einer weiteren chemischen Reaktion wurden die beiden Gruppen zur Ausbildung einer kovalenten Bindung verwendet, die dann das Protein beständig mit dem DNA Oligonukleotid verbindet. Die unbeteiligten Reaktionspartner wurden entfernt, und das so markierte Protein wurde in den folgenden Nachweisreaktionen eingesetzt.

In dieser Versuchsreihe wurde für Protein BSA und für die DNA Codes das Oligo 570 5'TTTTTTTTTTGAATGATATGCGACGCGTG 3' verwendet.

Nach der Markierungsreaktion des BSAs mit dem Oligo 570 wurde die Bindungseigenschaft der Protein-DNA Chimäre an das reverse komplementäre Oligo 571 5'TTTTTTTTTTCACGCGTCGCATATCATTC 3' und auf ein nicht komplementäres Oligo 572
5'TTTTTTTTTTCATGCATCGTATGTCGTAA 3' getestet. Diese wurde als Array-Anteil (Spotreihe) auf einen festen Träger (z.B. hybcell) immobilisiert. Die Inkubation beinhaltete folgende Schritte:
- Lösen der Protein-DNA Chimäre in Inkubationspuffer bestehend aus PBS und 1% Casein
- Inkubation des festen Trägers bei 45°C (das sind 5°C unter der ermittelten Schmelztemperatur der kodierenden Sequenz) für 15min
- Waschen des Trägers zur Entfernung von ungebundenem Material mit PBS
- Inkubation des Trägers mit fluoreszenzmarkiertem anti-BSA Antikörper
- Waschen des Trägers zur Entfernung von ungebundenem Material mit PBS
- Scannen der Trägeroberfläche und Ermittlung der Signalintensitäten

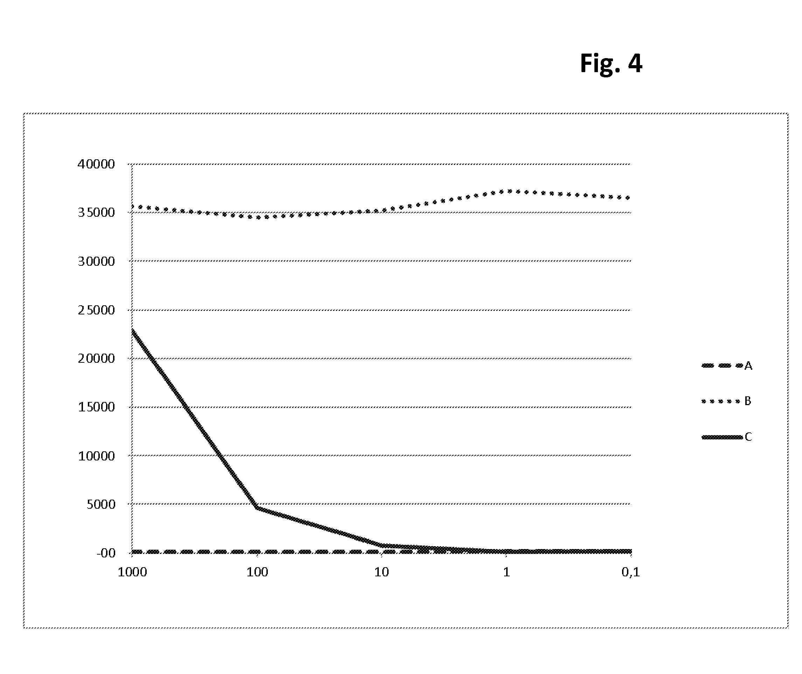

Das Ergebnis führte zu einer klaren Diskriminierung der verschiedenen Messpunkte (Spots) aufgrund der Hybridisierungseigenschaften. Weiters wurde die Konzentrationsabhängigkeit gemessen und in Fig. 4 zusammengefasst. D.h. die Anwendung des erfindungsgemäßen Verfahrens ist u.a. ideal für die Zuordnung von Signalen bei der parallelen Vermessung mehrerer Proben mit einem Festphasen Array (z.B. hybcell) geeignet.

## Patentansprüche

1. Verfahren zum parallelen Nachweis mindestens eines spezifischen Nukleinsäurezielmoleküls in einer Mehrzahl an Proben mittels Nukleinsäureamplifikation umfassend die Schritte:
a) Bereitstellen von mindestens zwei Nukleinsäure umfassenden Proben,
b) Kontaktieren der Proben aus Schritt a) mit einem Vorwärtsprimer und einem Rückwärtsprimer umfassend einen 3'- und 5'-Abschnitt, wobei der Vorwärts- und der Rückwärtsprimer im 3'-Abschnitt eine zum amplifizierenden Nukleinsäurezielmolekül komplementäre Nukleotidsequenz aufweisen und der Vorwärts- oder der Rückwärtsprimer im 5'-Abschnitt ein Oligonukleotid mit einer künstlichen, den einzelnen Proben zugeordneten Nukleinsäuresequenz umfasst,
c) Amplifizieren der Proben aus Schritt b),
d) Kontaktieren der amplifizierten Proben aus Schritt c) mit einem festen Träger, auf welchem an einem vorselektierten Ort Oligonukleotide immobilisiert sind, welche an deren 3'-Ende die künstliche, den einzelnen Proben zugeordnete Nukleinsäuresequenz umfassen, und
e) Feststellen der Bindung der Amplifikationsprodukte aus Schritt c) an den am festen Träger immobilisierten Oligonukleotiden.

2. Verfahren nach Anspruch 1, **dadurch gekennzeichnet, dass** der Träger planar oder rotationssymmetrisch ausgeformt ist.

3. Verfahren nach Anspruch 2, **dadurch gekennzeichnet, dass** der rotationssymmetrische Träger aus einem unter Freilassung eines radialen Ringspaltes in einen Probenbehälter einsetzbaren Rotor besteht, dessen Umfangsfläche mit den Oligonukleotiden immobilisiert ist.

4. Verfahren nach einem der Ansprüche 1 bis 3, **dadurch gekennzeichnet, dass** die Oligonukleotide mit der künstlichen, den einzelnen Proben zugeordneten Nukleinsäuresequenz eine Länge von 15 bis 30, vorzugsweise 18 bis 25, noch mehr bevorzugt von 19 bis 23, Basenpaaren aufweisen.

5. Verfahren nach einem der Ansprüche 1 bis 4, **dadurch gekennzeichnet, dass** die Oligonukleotide mit der künstlichen, den einzelnen Proben zugeordneten Nukleinsäuresequenz einen Schmelzpunkt zwischen 50 und 70°C, vorzugsweise ungefähr 60°C, aufweisen.

6. Verfahren nach einem der Ansprüche 1 bis 5, **dadurch gekennzeichnet, dass** die Rückwärtsprimer mit einem Farbstoff markiert sind.

7. Verfahren nach einem der Ansprüche 1 bis 5, **dadurch gekennzeichnet, dass** die Amplifikationsprodukte nach Schritt c) oder d) mit einem Farbstoff markiert werden.

8. Verfahren nach Anspruch 6 oder 7, **dadurch gekennzeichnet, dass** der Farbstoff ausgewählt ist aus der Gruppe bestehend aus Alexa Fluor, insbesondere Alexa Fluor 350, Alexa Fluor 488, Alexa Fluor 430, Alexa Fluor 555, Alexa Fluor 610 und Alexa Fluor 647, Bodipy (boron-dipyrromethene), insbesondere Bodipy 493/503, Bodipy R6G-X, Bodipy 530/550, Bodipy 558/568, Bodipy 564/570, Bodipy TMR-X, Bodipy 576/589, Bodipy 581/591, Bodipy TR-X, Bodipy 630/650, Bodipy 650/665 und Bodipy FL, Oregon Green, insbesondere Oregon Green 488, Oregon Green 500 und Oregon Green 514, FAM, FITC, Fluorescein, Fluorescein-dT, Rhodamin Green, TET, JOE, Yakima Yellow, HEX, CY3, TAMRA, Rhodamin Red, CY3.5, ROX, Texas Red, CY5, CY5.5, IRD700 und IRD800.

9. Verfahren nach einem der Ansprüche 1 bis 8, **dadurch gekennzeichnet, dass** die am festen Träger gebundenen Amplifikationsprodukte einer Primer-Verlängerung unterzogen werden.


SEQUENCE LISTING

<110>  Anagnostics Bioanalysis GmbH

<120>  Verfahren zum Nachweis von Nukleinsäuren

<130>  R 56129

<160>  3

<170>  PatentIn version 3.5

<210>  1
<211>  29
<212>  DNA
<213>  Artificial Sequence

<220>
<223>  Synthetic Oligonucleotide

<400>  1
tttttttttt gaatgatatg cgacgcgtg                                          29


<210>  2
<211>  29
<212>  DNA
<213>  Artificial Sequence

<220>
<223>  Synthetic Oligonucleotide

<400>  2
tttttttttt cacgcgtcgc atatcattc                                          29


<210>  3
<211>  29
<212>  DNA
<213>  Artificial Sequence

<220>
<223>  Synthetic Oligonucleotide

<400>  3
tttttttttt catgcatcgt atgtcgtaa                                          29